Europäisches Patentamt

(19) **European Patent Office**

Office européen des brevets

(11) Veröffentlichungsnummer: **0 037 895**
**B1**

(12) **EUROPÄISCHE PATENTSCHRIFT**

(45) Veröffentlichungstag der Patentschrift:
**07.03.84**

(51) Int. Cl.³: **C 07 C 23/02,** C 07 C 17/02

(21) Anmeldenummer: **81101467.9**

(22) Anmeldetag: **28.02.81**

(54) **Verfahren zur Herstellung von Hexabromcyclododecan gleichbleibender Qualität.**

(30) Priorität: **03.04.80 DE 3013002**

(43) Veröffentlichungstag der Anmeldung:
**21.10.81 Patentblatt 81/42**

(45) Bekanntmachung des Hinweises auf die Patenterteilung:
**07.03.84 Patentblatt 84/10**

(84) Benannte Vertragsstaaten:
**FR GB IT NL**

(56) Entgegenhaltungen:
**DE - B - 1 222 049**
**DE - B - 1 290 543**

(73) Patentinhaber: **Chemische Fabrik Kalk GmbH, Kalker Hauptstrasse 22 Postfach 91 01 57, D-5000 Köln 91 (DE)**

(72) Erfinder: **Rabe, Otto, Olpener Strasse 61, D-5000 Köln 91 (DE)**
Erfinder: **Jenkner, Herbert, Dr. Dipl. Chem., Am Quenchenhauf 8, D-5024 Pulheim 2 (DE)**

Verfahren zur Herstellung von Hexabromcyclododecan gleichbleibender Qualität

Hexabromcyclododecan ist u.a. aufgrund seines hohen Bromgehalts ein bewährter Zusatzstoff zur Brandschutzausrüstung von Kunststoffen oder Kunststoffschäumen, wie beispielsweise Polystyrolschaum. In vielen Fällen ist es notwendig, trotz eines Brandschutzzusatzes ein möglichst farbneutrales Polymerisat zu erhalten. Das setzt voraus, dass solche eingearbeitete Zusatzstoffe nicht gefärbt sind und möglichst keine farbverändernden Verunreinigungen enthalten. Vielfach entstehen derartige Verfährbungen jedoch, wenn an sich farblose bromhaltige Brandschutzmittel mit einem geringen Gehalt ebenfalls farbloser Verunreinigungen bei der Einarbeitung in Kunststoffe hohen Temperaturen ausgesetzt werden.

Hexabromcyclododecan kann in Form farbloser Kristalle nach einem in der DE-PS Nr. 1290543 beschriebenen Verfahren hergestellt werden. Nach diesem Verfahren wird Cyclododecatrien-1,5,9 in einem vorgelegten polaren Lösungsmittel, vorzugsweise Äthanol, bei niedrigen Temperaturen, vorzugsweise 10 bis 40° C, mit dem gleichzeitig zugefügten Brom umgesetzt. Vorteilhaft wird dabei während der Reaktion ein Bromüberschuss von etwa 5 Gew.-% aufrechterhalten. Das hierbei entstandene Hexabromcyclododecan scheidet sich als rein weisses kristallines Produkt schon nach kurzer Zeit ab und kann sofort nach dem Trocknen seinem Verwendungszweck zugeführt werden.

Die bei dieser Verfahrensweise anfallende Mutterlauge lässt sich für nachfolgende Verfahrensabläufe wieder verwenden, nur wird jedoch das hierbei anfallende Hexabromcyclododecan bei jeder Wiederverwendung der Mutterlauge zunehmend mit Verunreinigungen durchsetzt, die sich in der Mutterlauge anreicherten. Nach achtmaliger Wiederverwendung der Mutterlauge ist das Hexabromcyclododecan so stark gefärbt, dass die Lichtdurchlässigkeit, die anfangs etwa 60% beträgt, auf etwa 5% herabgesunken ist. Ein solches Hexabromcyclododecan ist für viele Anwendungszwecke nicht mehr geeignet, da sein Schmelzbereich nur noch zwischen 148 und 175° C liegt.

Es ergab sich die Aufgabe, Hexabromcyclododecan bei unbegrenzter Wiederverwendung der Reaktionslösung in gleichbleibender Qualität herzustellen.

Es wurde ein Verfahren zur Herstellung von Hexabromcyclododecan von gleichbleibender Qualität durch Umsetzung von Cyclododecatrien-1,5,9 mit Brom in einem polaren Lösungsmittel bei niedriger Temperatur unter Wiederverwendung der Reaktionslösung gefunden. Danach wird ein Teil der nach Abtrennung des hexabromcyclododecans verbleibenden Mutterlauge ausgeschleust, durch reines Lösungsmittel ersetzt und dieses Gemisch als Reaktionsmedium in den Verfahrensablauf zurückgeführt. Es hat sich besonders bewährt, nach Abtrennung des Hexabromcyclododecans 10 bis 50, vorzugsweise 20 bis 35 Gew.-% der Mutterlauge durch reines Lösungsmittel, vorzugsweise Äthanol, zu ersetzen.

Das Verfahren der Erfindung kann auch kontinuierlich betrieben werden, wenn beispielsweise Lösungsmittel im Gemisch mit Hexabromcyclododecan aus dem Reaktionsgefäss im Nebenschluss ausgetragen und das Hexabromcyclododecan hieraus abgetrennt wird. Das ausgetragene Lösungsmittel beträgt dabei 10 bis 50 Gew.-% der Gesamtmenge an Lösungsmittel, das durch Zusatz von reinem Lösungsmittel gemäss der Erfindung ersetzt wird.

Hexabromcyclododecan wird in einem ersten Ansatz nach dem in der DE-PS Nr. 1290543 beschriebenen Verfahren hergestellt, indem einer vorgelegten Menge Äthanol durch zwei örtlich voneinander getrennte Dosiervorrichtungen Cyclododecatrien und Brom bei einer Temperatur von etwa 10 bis 30° C zugefügt werden. Für 162 Gew.-Teile Cyclododecatrien werden etwa 400 bis 1000 Gew.-Teile Reaktionsmedium benötigt. Die nach beendeter Reaktion erhaltene Mutterlauge ist die Grundlage für das vorliegende erfindungsgemässe Verfahren.

Für das erfindungsgemässe Verfahren werden beispielsweise 76 Gew.-% der Mutterlauge, die noch etwa 0,8 bis 1,5 Gew.-% Brom enthält, mit etwa 24 Gew.-% wasserfreiem Äthanol vermischt. Dieses Gemisch wird für den Verfahrensablauf als Reaktionsmedium eingesetzt und ihm entsprechend dem vorangegangenen Ansatz Cyclododecatrien-1,5,9 und die diesem äquimolaren Menge Brom zuzüglich 5 Gew.-% Überschuss aus zwei getrennten Dosiervorrichtungen unter Rühren zugefügt. Die Reaktionstemperatur soll dabei nicht über 25° C ansteigen. Nach einer 1stündigen Nachreaktionszeit liegt in dem Reaktionsgemisch ein Überschuss an unverbrauchtem Brom von etwa 10 bis 15 Gew.-Teilen vor. Die Menge des nicht verbrauchten Broms hängt im hohen Masse von der Qualität des eingesetzten Brom bzw. des eingesetzten Cyclododecatriens ab und kann daher auch zwischen 3 bis 20 Gew.-Teilen variieren.

Nach beendeter Reaktion wird das entstandene Hexabromcyclododecan abgetrennt und mit wasserfreiem Äthanol nachgewaschen. Von der abgetrennten Mutterlauge werden 76 Gew.-% mit 24 Gew.-% wasserfreiem Äthanol vermischt und in diesem Mischungsverhältnis als Reaktionsmedium für einen nachfolgenden Ansatz wieder verwendet. Diese Wiederverwendung lässt sich beliebig oft fortsetzen.

Das erhaltene Hexabromcyclododecan wird mit Wasser aufgeschlämmt und letzte Reste von anhaftendem Brom mit Alkali oder Ammoniak entfernt, anschliessend neutral gewaschen und bei einer Temperatur von etwa 75° C getrocknet.

Die Menge der ausgeschleusten und nicht wiederverwendeten Mutterlauge hängt im hohen Masse von der Qualität des eingesetzten Broms bzw. des eingesetzten Cyclododecatriens ab. Aber auch der spätere Verwendungszweck des hexabromcyclododecans, vornehmlich als Brandschutzmittel für Kunststoffe bzw. Kunststoffschäume, wie beispielsweise Polystyrolschaum, bestimmt in gewissem Masse die Menge der ausgeschleusten Mutterlauge bzw. den neu zugefügten Anteil an reinem Lösungsmittel. Wird beispielsweise ein Hexabromcyclododecan von besonderer Reinheit und hohem Schmelzpunkt gewünscht, und wird aber hingegen nur ein Brom von minderer Qualität eingesetzt, oder es wird ein Cyclododecatrien, das infolge längerer Lagerungszeit nicht mehr seine ursprüngliche Qualität aufweist, verwendet, muss mehr Mutterlauge ausgeschleust werden. Im allgemeinen hat es sich jedoch gezeigt, dass bei einer Ausschleusung von 10 bis 50, vorzugsweise 24 bis 35 Gew.-% der Lösungsmittelmenge Hexabromcyclododecan von hoher Reinheit und hoher Ausbeute erhalten wird. Selbstverständlich kann die Menge der ausgeschleusten Mutterlauge nach Belieben vergrössert und durch Zugabe einer entsprechenden Menge an reinem Lösungsmittel ersetzt werden.

Das nach dem erfindungsgemässen Verfahren hergestellte Hexabromcyclododecan zeigt in 30%iger Dimethylformamidlösung bei einer Schichtdicke von 2,001 cm und einem Filter S 42 E 68 eine Lichtdurchlässigkeit von etwa 81%. Dieser Wert von 80 bis 90% Lichtdurchlässigkeit bleibt auch nach 40maliger Wiederverwendung der Mutterlauge konstant.

In dem folgenden Beispiel soll das erfindungsgemässe Verfahren näher erläutert werden.

*Beispiel*

1286 Gew.-Teile Mutterlauge, die noch 10 bis 20 Gew.-Teile Brom enthält, wird mit 395 Gew.-Teilen wasserfreiem Äthanol aufgefüllt und in einem Reaktionsgefäss vorgelegt. Bei einer Temperatur von 25° C werden 60 Gew.-Teile Brom, entsprechend einem Überschuss von 5 Gew.-%, zugeführt. Unter Rühren und gegebenenfalls unter Kühlung werden aus zwei örtlich voneinander getrennten Dosiervorrichtungen gleichzeitig 1198 Gew.-Teile Brom und 387 Gew.-Teile Cyclododecatrien-1,5,9, im Laufe von 10 h so zugeführt, dass die Reaktionstemperatur 25° C nicht übersteigt. Die Zudosierung von Brom und Cyclododecatrien wird so eingestellt, dass ein gleichbleibendes Mengenverhältnis in beiden Dosiervorrichtungen während der Reaktion aufrechterhalten wird, d.h. ein Verhältnis von 3 Gew.-Teilen Brom zu 1 Gew.-Teil Cyclododecatrien besteht. Nach 1stündiger Nachreaktion wird das schon nach kurzer Zeit ausfallende Hexabromcyclododecan abfiltriert und mit 315 Gew.-Teilen wasserfreiem Äthanol gewaschen, das dann der Reaktionslösung zugefügt wird. Es werden nach dem Abfiltrieren insgesamt 1972 Gew.-Teile Mutterlauge erhalten mit einem Gehalt an unverbrauchtem Brom von 12 Gew.-Teilen. 1286 Gew.-Teile dieser Mutterlauge werden wiederum mit 395 Gew.-Teilen Äthanol vermischt und für einen folgenden Ansatz wieder verwendet. Aus der ausgeschleusten Mutterlauge kann der Alkohol in an sich bekannter Weise wiedergewonnen und als Lösungsmittel für weitere Ansätze verwendet werden.

Das mit Äthanol gewaschene Hexabromcyclododecan wird zur Entfernung von anhaftenden Verunreinigungen bzw. der letzten Reste Brom in 1400 Gew.-Teilen 2%iger Natriumhydroxidlösung suspendiert und anschliessend neutral gewaschen. Nach dem Trocknen bei 75° C werden 1462 Gew.-Teile farbloses kristallines Hexabromcyclododecan mit einem Wassergehalt von weniger als 0,5% erhalten. Die Ausbeute beträgt 95% der Theorie, bezogen auf eingesetztes Cyclododecatrien. Die Analyse weist einen Bromgehalt von 73,7% aus, der Schmelzbereich liegt bei 180-190° C. Die Lichtdurchlässigkeit, festgestellt mit einer 30 gew.-%igen Lösung des Hexabromcyclododecans in Dimethylformamid in einer Schichtdicke von 2,001 cm in einer Quarzküvette und einem Filter S 42 E 68 (= 420 nm), beträgt 81%.

Das Verfahren gemäss diesem Beispiel wird achtmal wiederholt, der Bromgehalt und der Schmelzbereich bleiben praktisch konstant, die Lichtdurchlässigkeit liegt zwischen 80 und 90%. Auch nach 40-facher Wiederholung kann keine Veränderung der physikalischen Daten festgestellt werden.

**Patentansprüche**

1. Verfahren zur Herstellung von Hexabromcyclododecan von gleichbleibender Qualität durch Umsetzung von Cyclododecatrien-1,5,9 mit Brom in einem polaren Lösungsmittel bei niedriger Temperatur unter Wiederverwendung der Reaktionslösung, dadurch gekennzeichnet, dass ein Teil der nach Abtrennung des Hexabromcyclododecans verbleibenden Mutterlauge ausgeschleust und durch reines Lösungsmittel ersetzt wird und dieses Gemisch als Reaktionsmedium in den Verfahrensablauf zurückgeführt wird.

2. Verfahren nach Anspruch 1, dadurch gekennzeichnet, dass 10 bis 50% der Mutterlauge ausgeschleust werden.

3. Verfahren nach den Ansprüchen 1 und 2, dadurch gekennzeichnet, dass als polares Lösungsmittel Äthanol verwendet wird.

**Revendications**

1. Procédé de préparation d'hexabromocyclododécane de qualité constante par mise en réaction de cyclododécatriène-1,5,9 avec du brome dans un solvant polaire à basse température avec réutilisation de la solution réactionnelle, caractérisé en ce qu'une partie de la liqueur mère restant après séparation de l'hexabromocyclododécane est éclusée et remplacée par du solvant pur, ce mélange étant réintroduit dans le processus en tant que milieu réactionnel.

2. Procédé suivant la revendication 1, caractérisé en ce que 10 à 50% de la liqueur mère est éclusé.

3. Procédé suivant les revendications 1 et 2, caractérisé en ce que le solvant polaire utilisé est de l'éthanol.

## Claims

1. Process for the preparation of hexabromocyclododecane of constant quality by reacting cyclododecatriene-1,5,9 with bromine in a polar solvent at low temperature with re-use of the reaction solution, characterized in that a portion of the mother liquor remaining after separation of the hexabromocyclododecane is sluiced and replaced with pure solvent, this mixture being then refed into the process as reaction medium.

2. Process according to claim 1, characterized in that 10 to 50% of the mother liquor is sluiced.

3. Process according to claims 1 and 2, characterized in that ethanol is used as the polar solvent.